(19) Europäisches Patentamt
European Patent Office
Office européen des brevets

(11) **EP 4 423 762 B1**

(12) **EUROPEAN PATENT SPECIFICATION**

(45) Date of publication and mention
of the grant of the patent:
**22.10.2025 Bulletin 2025/43**

(21) Application number: **22809041.1**

(22) Date of filing: **24.10.2022**

(51) International Patent Classification (IPC):
**G16H 20/17** (2018.01)     **G16H 40/63** (2018.01)
**A61M 5/172** (2006.01)

(52) Cooperative Patent Classification (CPC):
**G16H 20/17; G16H 40/63;** A61M 5/1723

(86) International application number:
**PCT/EP2022/079500**

(87) International publication number:
**WO 2023/072789 (04.05.2023 Gazette 2023/18)**

(54) **SYSTEM AND METHOD FOR CONTROLLING A TARGET-CONTROLLED INFUSION**

SYSTEM UND VERFAHREN ZUR STEUERUNG EINER ZIELGESTEUERTEN INFUSION

SYSTÈME ET PROCÉDÉ DE COMMANDE D'UNE PERFUSION COMMANDÉE PAR LA CIBLE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **29.10.2021 EP 21315230**

(43) Date of publication of application:
**04.09.2024 Bulletin 2024/36**

(73) Proprietor: **Fresenius Vial SAS
38590 Brézins (FR)**

(72) Inventors:
• **TERRIER, David
38590 Sillans (FR)**
• **BARBEYRAC, Damien
38113 Veurey Voroize (FR)**
• **ARTARIT, Olivier
38960 Saint Etienne de Crossey (FR)**

(74) Representative: **Fresenius Kabi Deutschland
GmbH
Patent Department - MedTech
Else-Kröner-Straße 1
61352 Bad Homburg (DE)**

(56) References cited:
**EP-A1- 3 228 343     US-A1- 2018 085 520
US-B1- 10 842 932**

**Description**

The invention relates to a system for controlling a target-controlled infusion for administering a drug to a patient according to the preamble of claim 1 and to a method for controlling a target-controlled infusion for administering a drug to a patient.

A system of this kind comprises at least one infusion device for administering a drug to the patient and a control device configured to control operation of the at least one infusion device. The control as performed by the control device herein is such that a drug concentration e.g. at an effect site within the patient is established which is at or at least close to a target concentration, wherein the target concentration may be constant over a period of time, or may vary such that a concentration within the patient is controlled to follow a certain concentration curve. Herein, the control device is configured to execute a target-controlled infusion protocol using a mathematical model modeling a drug distribution in the patient's body for controlling operation of the at least one infusion device.

"Target-controlled infusion" (TCI) generally refers to an infusion operation performed by an computer-assisted infusion system which calculates a substance concentration in a particular body compartment on the basis of a mathematical model and which, after setting a target concentration, adjusts the infusion rate such that the concentration in the body compartment of the patient converges towards and is kept at the predefined target concentration. TCI infusion systems generally consist of one or multiple infusion devices and a control device, which may be separate to the infusion devices or may be integrated into an infusion device.

For setting up an infusion operation, herein, patient specific parameters such as the patient's age, weight, gender, and drug specific parameters such as the type of drug, e.g. the type of anesthetic, and a desired target concentration in a body compartment of the patient, for example relating to a drug level in the patient's brain within an anesthesia procedure, may be entered into the system using a human machine interface. In addition, a suitable mathematical model, such as a pharmacokinetic/pharmacodynamic model out of a multiplicity of models defined in the system, may be chosen for executing a target-controlled infusion protocol. When performing a target-controlled infusion operation, then, the control device executes the target-controlled infusion protocol and in this context calculates infusion rates in order to control one or multiple infusion devices for administering one or multiple specified drugs to the patient.

On the basis of an empirically determined population-pharmacokinetic model and using a known pharmacokinetic and patient-specific pharmacodynamic parameter set of a medicament (for example propofol) as well as by means of patient-specific data, a TCI system models a drug distribution (over time) within the patient's body by calculating drug concentrations in body compartments as defined within the model. During the execution of a target-controlled infusion protocol, herein, the mathematical model may be repeatedly adjusted according to measurement values relating to a drug concentration within the patient, for example by measuring a drug concentration in a patient's breath or in the patient's plasma (blood) compartment, or by measuring biological signals such as EEG or ECG signals or by deriving indices such as the so-called bi-spectral (BIS) index. According to measurement values the mathematical model is used during operation such that it suitably reflects the concentrations in the patient's body compartments according to the measurement values, such that patient-individual effects such as a patient-specific metabolism may be taken into account. The mathematical model may hence accurately model the drug concentration within the body, which may be used to control the infusion operation using one or multiple infusion devices in order to set or maintain a desired concentration e.g. in a desired effect site compartment within the patient to obtain a desired medical effect, such as an anesthetic effect during an anesthesia procedure.

Systems and methods for performing target-controlled infusion operations, in particular anesthetic operations, are for example known from EP 1 418 976 B1, WO 2016/160321 A1, and WO 2017/190966 A1. US 10 842 932 B1 relates to the titration and delivery of anesthetic and sedative medications to a subject. US 2018/085520 A1 describes an infusion mode for an infusion pump configured for the scheduling of infusions without requiring the clinician to hand-calculate and manually update the infusion pump, should a stoppage occur, such as a pump occlusion. The infusion pump comprises a control module configured to receive within-time infusion mode pump parameters.

When administering a drug to a patient using an infusion device, the infusion device generally is connected to the patient by means of an infusion line. Herein, in certain events an occlusion may occur in the infusion line, which may hinder the delivery of the drug towards the patient and hence may represent an obstacle to the infusion operation, potentially having severe consequences for the patient. For this reason, the infusion device comprises a sensor device configured to measure a value indicative of a pressure in the infusion line, such that by observing said value and in particular a rise in the value indicative of the pressure in the infusion line an occlusion may be detected. If an occlusion is detected, a warning message may be triggered and the infusion may be halted, such that a user is enabled to release the occlusion in the infusion line.

EP 3 228 343 A1 describes a system and method for operating an infusion device for administering a medical fluid to a patient. Herein, by observing a pressure in the infusion line an occlusion may be detected, and in case an occlusion is detected a pumping mechanism of the infusion device is operated in a reverse manner in order to reduce the pressure in the infusion line. In this way an undesired bolus in case of an occlusion release can be avoided.

Generally, if an occlusion in an infusion line occurs at a certain point in time, the occlusion will not be detected immediately, but only after lapse of some time, due to the fact that the occlusion is generally detected by observing the pressure in the infusion line and, in case of an occlusion, the pressure in the infusion line builds up continuously until a predefined threshold is exceeded. An occlusion hence may detected only a significant amount of time after the true occurrence of the occlusion, possibly in the range of multiple tens of minutes or even hours.

If the infusion operation is controlled by applying a target-controlled infusion protocol, the occurrence of an occlusion may cause the mathematical model to model the drug distribution in the patient's body based on the assumption that the drug is continuously delivered to the patient even after the occlusion occurs (but is not yet detected), leading to an incorrect modeling of the drug distribution in the patient's body.

It is an object of the instant invention to provide a system and a method which allow for a correct modeling of a drug distribution in the patient's body even in case an occlusion in an infusion line occurs.

This object is achieved by means of a system comprising the features of claim 1.

Accordingly, the control device is configured to store, at each of a multiplicity of points in time during execution of said target-controlled infusion protocol, a set of parameters derived from said mathematical model in a memory, wherein the control device is configured, in case an occlusion is detected, to use the set of parameters of a point in time prior to the detection of the occlusion in the mathematical model to model the drug distribution in the patient's body based on the set of parameters of the point in time prior to the detection of the occlusion.

In case an occlusion in an infusion line occurs during administration of a drug, from the time of the actual occlusion no drug or a very limited amount of drug is delivered through the infusion line to the patient. The detection of the occlusion herein takes place a significant amount of time after the actual occurrence of the occlusion, such that for the time period in between the actual occurrence of the occlusion and the detection of the occlusion no drug reaches the patient. However, during this time the infusion device is operated to pump a drug volume, causing a rise of pressure in the infusion line.

In the time between the actual occurrence of the occlusion and the detection of the occlusion the occlusion is not yet known, such that the mathematical model assumes that the volume of drug transported by the infusion device reaches the patient. The mathematical model hence models the drug distribution in the patient's body under the assumption that the drug volume transported by the infusion device is delivered to the patient. As the volume in fact does not reach the patient, the modeling of the drug distribution becomes inaccurate.

For this reason, it herein is proposed to store, at a multiplicity of points in time, parameters associated with the mathematical model as derived from the mathematical model, such as concentration values, mass values of compartments as used e.g. in a pharmacokinetic/pharmacodynamic model, parameters relating to transfer rates, parameters relating to an infused volume, and parameters relating to an infusion rate. Hence, during execution of the target-controlled infusion protocol information is repeatedly stored, e.g. providing for an actual image of the mathematical model at the different points in time.

This allows to revert to a previous set of parameters in case an occlusion is detected, such that the drug distribution in the patient's body may be modeled anew starting from a time prior to the detection of the occlusion. As it is known that at the time of detecting an occlusion a significant amount of time may have passed since the occurrence of the actual occlusion, based on the stored sets of parameters at multiple previous points in time it may be reverted to a set of parameters to be used in the mathematical model valid at a point in time at or close to the time of the actual occurrence of the occlusion.

If for example, based on the time of detection of the occlusion and based on an estimated volume that has given rise to an increase in pressure in the infusion line due to the presence of the occlusion, the time of the occurrence of the actual occlusion can be estimated, it may be reverted to that set of parameters at that point in time which is at or close to the time of the actual occurrence of the occlusion.

By using the set of parameters in the mathematical model, the mathematical model hence may be reset to the time of the actual occurrence of the occlusion, and based on the use of the set of parameters in the mathematical model the distribution of the drug in the patient's body may be re-calculated starting from the time of the actual occurrence of the occlusion. If, herein, in the mathematical model it is assumed that from the time of the occurrence of the occlusion no drug reaches the patient any longer, the mathematical model may take into account the decay of the drug concentrations and distribution in the patient's body following the occurrence of the occlusion, such that the actual drug distribution in the patient's body at and subsequent to the time of detecting the occlusion may correctly be modeled.

The control device, in one embodiment, is a separate device to the infusion device. The control device herein may serve to control operation of one or multiple infusion devices.

In another embodiment, the control device may be incorporated into an infusion device, such that the control device may be implemented by the infusion device and hence does not necessarily form a separate entity with respect to the at least one infusion device.

Further, the control device may be embodied by multiple entities. For example, a portion of the control device may be implemented by the infusion device, i.e., a processor off the infusion device, wherein another portion of the control device may be implemented by a device separate to the infusion device. For example, an occlusion detection may take place directly in an infusion device, whereas a control of the infusion using a target-controlled infusion protocol may take place by a device separate to the infusion device.

In one embodiment, the control device is configured to associate the set of parameters with a timestamp indicative of a corresponding of said multiplicity of points in time, and to store the set of parameters together with the associated timestamp in the memory. The information as derived from the mathematical model hence is timestamped, such that, in case of an occurrence of an occlusion, it may be reverted to a particular set of parameters associated with a particular timestamp relating to the time of the actual occurrence of the occlusion.

In one embodiment, the control device is configured to update the information in the memory by overwriting the information in the memory stored at a point in time by updated information computed at a later point in time. Hence, information is repeatedly derived from the mathematical model by storing a set of parameters relating to the mathematical model, wherein not all information is maintained over all times, but current information is used to overwrite prior information, such that in case of a detection of an occlusion the most recent information and information at a predefined number of previous points in time (identified by associated timestamps) is available and can be used to search for the relevant set of parameters at the time of the actual occurrence of the occlusion.

For example, a number between 20 to 100, for example between 25 to 50 sets of parameters may be stored. If the maximum number of sets of parameters is reached, the oldest set of parameters in each case is overwritten by the latest set of parameters in the course of the infusion operation.

In one embodiment, the multiplicity of points in time are equidistantly spaced apart at a predefined time interval. Hence, at regular intervals information is derived and stored, the interval being chosen such that suitable information is available at any time.

In another embodiment, the points in time are not equidistantly spaced in time, but are for example associated each with a predefined change in an infused volume. For example, a set of parameters may be stored after an infusion step corresponding to a volume between 0.05 ml to 0.5 ml, for example 0.1 ml, such that in each case after the predefined volume has been delivered a set of parameters is stored.

In one embodiment, the control device is configured, at the time of detection of the occlusion, to initiate a pressure release process to reduce a pressure within the infusion line. The pressure release process may for example be carried out as it is described in EP 3 228 343 A1. For example, during the pressure release process a pump mechanism of the infusion device may be operated in a reverse pumping direction in order to reduce an amount of an infused volume which has been transported by the infusion device during the infusion operation.

For example, as described in EP 3 228 343 A1, the infusion device may be operated in a reverse direction in order to pump a volume in a reverse, upstream direction which is estimated to be equivalent to the volume which has given rise to the pressure increase in the infusion line. Namely, based on the pressure threshold employed for detecting the occlusion in the infusion line and a compliance of the infusion line the drug volume that has been transported by the infusion device after occurrence of the occlusion (that is the volume which is transported by the infusion device during the occlusion, which however has not reached the patient due to the occlusion in the infusion line) can be computed using the following equation:

$$V[ml] = C[ml / bar] \cdot P_{thres}[bar] \cdot K$$

Herein, V is the transported volume after occurrence of the occlusion, C is the compliance of the infusion line and/or a pumping mechanism (such as a syringe) of the infusion device, $P_{thres}$ is the pressure threshold used for the occlusion detection, and K is a constant factor (e.g. known by calibration).

As the transported volume indicates that volume which is transported by the infusion device after the actual occurrence of the occlusion, but which has not reached the patient due to the occlusion, the infusion device may be operated in a reverse direction in order to reduce an overall infused volume (i.e., the volume transported during the entire infusion operation) by a reduction volume corresponding to the transported volume after occurrence of the occlusion. In this way the pressure rise in the infusion line due to the occurrence of the occlusion is reversed.

The infused volume indicates that volume which has been transported by the infusion device during the entire infusion operation. The transported volume indicates that volume which is transported by the infusion device after the actual occurrence of the occlusion until the detection of the occlusion and which hence has not reached the patient during the occlusion. By reducing the infused volume by the reduction volume corresponding to the transported volume, the infused volume hence is reduced by that amount which has not reached the patient during the presence of the occlusion, but which has been trapped in the infusion line due to the occlusion. By the reverse pumping action the pressure in the infusion line due to the occlusion hence is released.

In one embodiment, the control device is configured to identify the set of parameters of the point in time prior to the detection of the occlusion based on the reduction volume. If for example the transported volume (which is transported by the infusion device after the occurrence of the actual occlusion until the detection of the occlusion) is computed as described above, based on the value for the transported volume the reduction volume can be determined, and based on the reduction volume it can be searched for that set of parameters prior to the detection of the occlusion which is associated with an infused volume (i.e. the total volume infused during the infusion operation) equal to the infused volume at the time of the detection of the occlusion minus the reduction volume. The mathematical model hence is reset to a state at which the volume transported during the occlusion is reverted, and hence approximately to the state at the actual occurrence of the occlusion.

In one embodiment, the control device may be configured to identify the set of parameters of the point in time prior to the detection of the occlusion by comparing a value indicative of the infused volume as stored in the sets of parameters of the multiplicity of points in time to a value indicative of the infused volume as a result of the pressure release process. For example, the control device may start its comparison with the most recent set of parameters, i.e., the set of parameters associated with the most recent point in time. From that point in time it may search backwards, such that it is searched for a set of parameters in which the infused volume as stored in the set of parameters becomes equal to or smaller than the value indicative of the infused volume as a result of the pressure release process.

The set of parameters in which the infused volume (i.e., the total volume infused during the infusion operation) is equal to or smaller than the infused volume at the end of the pressure release process corresponds to that state of the mathematical model at which approximately the occlusion has occurred. By using this set of parameters in the mathematical model and by modeling the drug distribution using this set of parameters, hence, the actual drug distribution in the patient's body during the occlusion may be computed, wherein it is assumed that during the occlusion no drug volume reaches the patient. Using the mathematical model, it hence may accurately be modeled how the drug distribution at the end of the pressure release process is, such that after releasing the pressure the modeling may continue with an accurate estimate of the drug distribution within the patient's body.

The mathematical model in particular may be a pharmacokinetic/pharmacodynamic model which models the drug distribution of a drug administered to a patient. Within the pharmacokinetic/pharmacodynamic model the drug concentration is modeled in different body compartments of a patient, in particular a plasma compartment, a brain compartment, a rapid equilibrating compartment (representative e.g. of muscle and inner organ tissue) and a slow equilibrating compartment (e.g. fat, bone tissue). The model herein may self-adjust during execution of the target-controlled infusion protocol in dependence on measurement values as obtained during execution, such that the model is individualized during execution and hence reflects patient-specific conditions as experienced during the target-controlled infusion operation. For this, in particular the control device may be configured to adjust at least a subgroup of a multiplicity of parameters of the mathematical model during execution of the target-controlled infusion protocol according to measurement values relating to a drug concentration distribution in the patient.

In another aspect, a method for controlling a target-controlled infusion for administering a drug to a patient comprises: controlling, using a control device, operation of at least one infusion device in order to establish a drug concentration within the patient based on a target concentration by executing a target-controlled infusion protocol using a mathematical model modelling a drug distribution in the patient's body for controlling operation of the at least one infusion device; measuring, using a sensor device of the at least one infusion device, a value indicative of a pressure in an infusion line connected to the at least one infusion device for delivering said drug to the patient; and detecting, using the control device, an occlusion in the infusion line connected to the at least one infusion device based on said value measured by the sensor device; storing, by the control device, a set of parameters derived from said mathematical model in a memory at each of a multiplicity of points in time during execution of said target-controlled infusion protocol; and in case an occlusion is detected, using, by the control device, the set of parameters of a point in time prior to the detection of the occlusion in the mathematical model to model the drug distribution in the patient's body based on the set of parameters of the point in time prior to the detection of the occlusion.

The advantages and advantageous embodiments as described above for the system equally apply also to the method, such that it shall be referred to the above in this respect.

The idea underlying the invention shall subsequently be described in more detail by referring to the embodiments shown in the figures. Herein:

Fig. 1 shows a schematic view of a setup of system for performing a target-controlled infusion (TCI);

Fig. 2 shows a functional diagram of the setup of Fig. 1;

Fig. 3 shows a functional diagram of a model for modelling the distribution of a drug dosage in a patient's body;

Fig. 4 shows a schematic diagram of a PK/PD model;

Fig. 5 shows a schematic diagram of another PK/PD model;

Fig. 6      shows a schematic view of a plasma compartment concentration and an effect site concentration converging towards a respective target concentration;

Fig. 7A      shows an example of an infusion rate during an infusion operation;

Fig. 7B      shows a schematic view of a plasma compartment concentration and an effect site concentration correlated with the infusion rate of Fig. 7A;

Fig. 8      shows a concentration curve, indicating the deriving of information relating to a mathematical model during the infusion operation;

Fig. 9A      shows an example of an infusion rate, in case of a detection of an occlusion and when carrying out a pressure release process in order to release a pressure in an infusion line as a result of the occlusion;

Fig. 9B      shows an infused volume as transported by an infusion device, correlated to the infusion rate of Fig. 9A;

Fig. 9C      shows an infused volume as it reaches the patient; and

Fig. 9D      shows an effect site concentration correlated to the infusion rate of Fig. 9A and the occurrence and release of the occlusion.

Subsequently, a system and method for administering one or multiple drugs to a patient in a target-controlled infusion (TCI) procedure, e.g. an anesthetic procedure, shall be described in certain embodiments. The embodiments described herein shall not be construed as limiting for the scope of the invention.

Like reference numerals are used throughout the figures as appropriate.

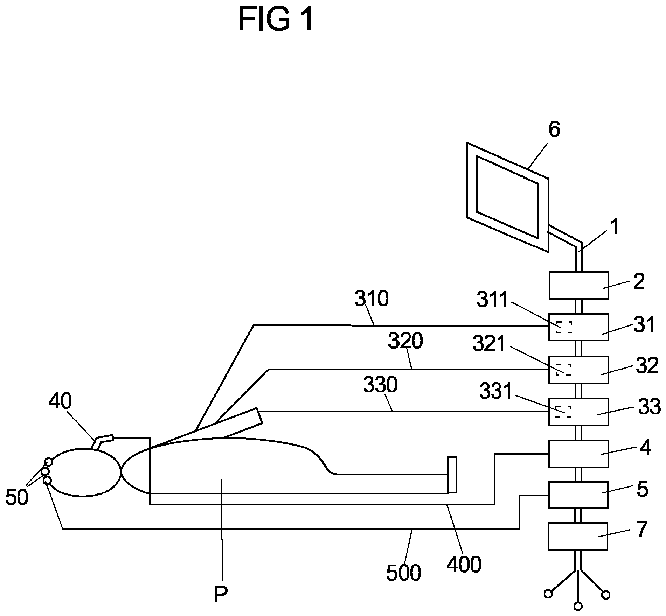

Fig. 1 shows a schematic drawing of a setup as it generally is used for example in an anesthesia procedure for administering anesthetic drugs, such as analgesic agents or hypnotic agents, e.g. propofol and/or remifentanil, to a patient P. In this setup multiple devices are arranged on a rack 1 and are connected via different lines to the patient P.

In particular, infusion devices 31, 32, 33 such as infusion pumps, in particular syringe pumps or volumetric pumps, are connected to the patient P and serve to intravenously inject, via lines 310, 320, 330, different drugs such as propofol, remifentanil and/or a muscle relaxant drug to the patient P in order to achieve a desired anesthetic effect. The lines 310, 320, 330 are for example connected to a single port providing access to the venous system of the patient P such that via the lines 310, 320, 330 the respective drugs can be injected into the patient's venous system.

The rack 1 furthermore may hold a ventilation device 4 for providing an artificial respiration to the patient P e.g. while the patient P is under anesthesia. The ventilation device 4 is connected via a line 400 to a mouth piece 40 such that it is in connection with the respiratory system of the patient P.

The rack 1 also holds a bio-signal monitor 5, for example an EEG monitor which is connected via a line or a bundle of lines 500 to electrodes 50 attached to the patient's head for monitoring the patient's brain activity e.g. during an anesthesia procedure.

In addition, a control device 2 is held by the rack 1 which serves to control the infusion operation of one or multiple of the infusion devices 31, 32, 33 such that infusion devices 31, 32, 33 inject drugs to the patient P in a controlled fashion to obtain a desired effect, e.g. an anesthetic effect. This shall be explained in more detail below.

It is to be noted herein that the control device 2 may also be incorporated into an infusion device 31, 32, 33, such that the control device 2 may be implemented by the infusion device 31, 32, 33.

Additional measurement devices may be used, e.g. for measuring the concentration of one or multiple drugs for example in the breath of the patient P or to measure information relating to and allowing to determine e.g. a bi-spectral index. A measurement device may for example be constituted by a so called IMS monitor for measuring a drug concentration in the patient's breath by means of the so called Ion Mobility Spectrometry. Other sensor technologies may also be used.

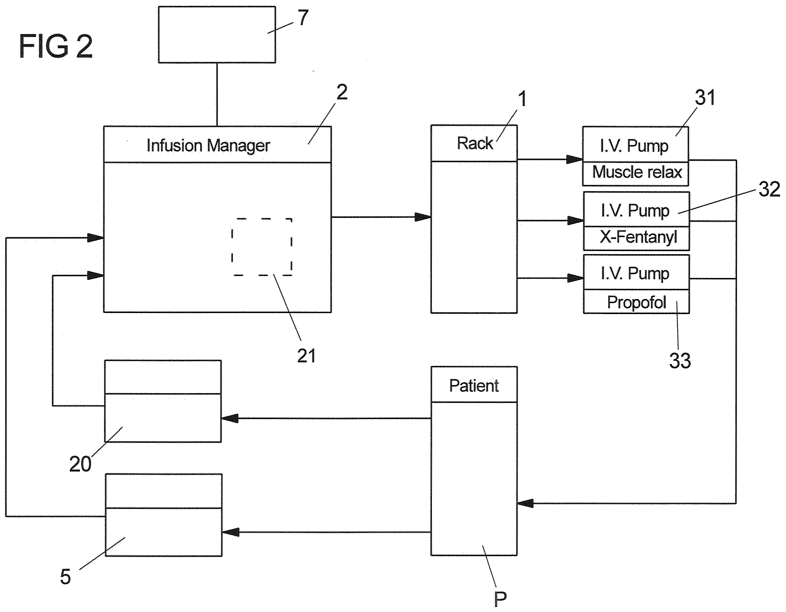

Fig. 2 shows a functional diagram of a control loop for controlling the infusion operation of infusion devices 31, 32, 33 during an infusion operation. The control loop herein may in principle be set up as a closed-loop in which the operation of the infusion devices 31, 32, 33 is automatically controlled without user interaction. Alternatively, the system is set up as an advisory (open-loop) system in which at certain points of time, in particular prior to administering a drug dosage to a patient, a user interaction is required in order to manually confirm the operation.

The control device 2, also denoted as "infusion manager", is connected to the rack 1 which serves as a communication link to the infusion devices 31, 32, 33 also attached to the rack 1. The control device 2 outputs control signals to control the operation of the infusion devices 31, 32, 33, which according to the received control signals inject defined dosages of drugs to the patient P.

By means of the bio-signal monitor 5, e.g. in the shape of an EEG monitor, for example an EEG reading of the patient P is taken, and by another measurement device 20 a concentration of one or multiple drugs in the patient's P breath is measured. The measured data are fed back to the control device 2, which correspondingly adjusts its control operation and outputs modified control signals to the infusion devices 31, 32, 33 to achieve a desired anesthetic effect.

The control device 2 uses, for controlling the infusion operation of one or multiple infusion devices 31, 32, 33, a pharmacokinetic-pharmacodynamic (PK/PD) model, which is a pharmacological model for modelling processes acting on a drug in the patient's P body. Such processes include the resorption, the distribution, the biochemical metabolism and the excretion of the drug in the patient's P body (denoted as pharmacokinetics) as well as the effects of a drug in an organism (denoted as pharmacodynamics). Preferably, a physiological PK/PD model with N compartments is used for which the transfer rate coefficients have been experimentally measured beforehand (for example in a proband study) and are hence known.

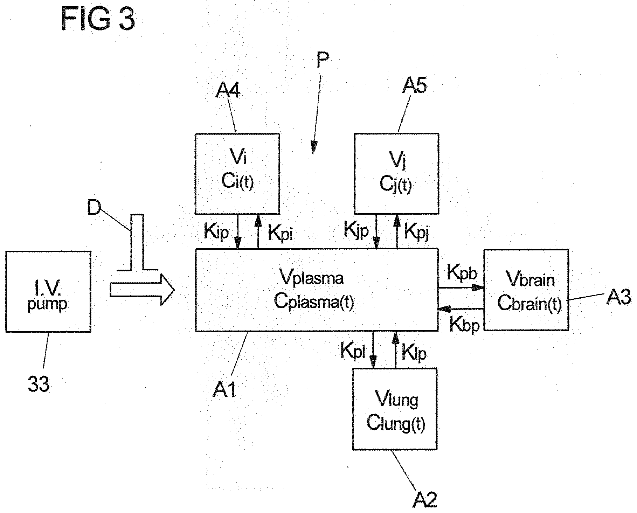

A schematic functional drawing of the setup of such a PK/PD model p is shown in Fig. 3. The PK/PD model p logically divides the patient P into different compartments A1-A5, for example a plasma compartment A1 corresponding to the patient's P blood stream, a lung compartment A2 corresponding to the patient's P lung, a brain compartment A3 corresponding to the patient's P brain and other compartments A4, A5 corresponding, for example, to muscular tissue or fat and connective tissue. The PK/PD model p takes into account the volume $V_{Lung}$, $V_{plasma}$, $V_{brain}$, $V_i$, $V_j$ of the different compartments A1-A5 as well as transfer rate constants $K_{PL}$, $K_{LP}$, $K_{BP}$, $K_{PB}$, $K_{IP}$, $K_{PI}$, $K_{JP}$, $K_{PJ}$ indicating the transfer rates between the plasma compartment A1 and the other compartments A2-A5, assuming that a drug dosage D by means on an infusion device 31-33 is injected into the plasma compartment A1 and the plasma compartment A1 links the other compartments A2-A5 such that an exchange between the other compartments A2-A5 always takes place via the plasma compartment A1. The PK/PD model p serves to predict the concentrations $C_{lung}$, $C_{plasma}$, $C_{brain}$, $C_i$, $C_j$ of the injected drug in the different compartments A1-A5 as a function of time.

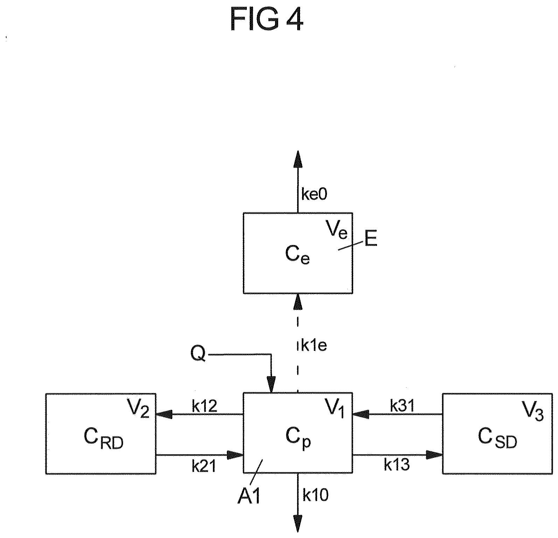

Fig. 4 illustrates, in a schematic diagram, an example of a PK/PD model which comprises a central plasma compartment A1 exhibiting a drug concentration $C_p$, a rapid equilibrating compartment exhibiting a drug concentration $C_{RD}$, a slow equilibrating compartment exhibiting a drug concentration $C_{SD}$, an dan effect compartment E comprising an effect compartment concentration $C_e$ of the drug. Herein,

$Q$      represents an administered drug,

$k_{e0}$      defines the proportional change in each unit of time of the concentration gradient between the plasma and effect-site,

$k_{1e}$      describes an elimination constant for redistribution of the drug from the effect compartment E to the plasma compartment A1,

$k_{12}$      is an elimination constant describing the distribution of the volume V1 in direction of volume V2,

$k_{21}$      is an elimination constant describing the distribution of the volume V2 in direction of volume V1,

$k_{13}$      is an elimination constant describing the distribution of the volume V1 in direction of volume V3,

$k_{31}$      is an elimination constant describing the distribution of the volume V3 in direction of volume V1,

$k_{10}$      represents the elimination constant of the applied drug from the body.

Fig. 4 herein visualizes a so called Schnider model. This assumes that after intravenous injection a drug Q is rapidly distributed in the circulation of the central plasma compartment A1 and quickly reaches well perfused tissues, such that a tissue-specific redistribution in various other compartments such as muscle or fat tissue occurs. At the same time the body eliminates the applied substance from the plasma compartment A1 with a certain elimination rate. For the pharmacokinetic characterization e.g. of lipophilic anesthetics, a 3-compartment model has been established that comprises a plasma compartment A1 (heart, lung, kidney, brain), a rapid equilibrating compartment exhibiting a concentration $C_{RD}$ (muscles, inner organs), and a slow equilibrating compartment exhibiting a concentration $C_{SD}$ (fat, bone, the so-called "deep" compartment). The concentration-time curve of a drug is characterized by the distribution volume of a specific compartment and the clearance (which is the plasma volume, from which the drug is eliminated per time unit): V1 denotes the volume of the plasma compartment A1, V2 is the volume of a well-perfused tissue $C_{RD}$ and V3 is the volume of a less perfused compartment, associated with a concentration $C_{SD}$. The clearance of a substance from the various compartments can be described by elimination constants. The elimination constant $k_{12}$ for example describes the distribution from the volume V1 towards the volume V2, and $k_{21}$ describes the distribution in the opposite direction. An applied substance is eliminated by this model with the constant $k_{10}$ from the body. After reaching an equilibrium ("steady state") between the individual compartments, the elimination rate determines the amount of substance that must be supplied to maintain equilibrium.

To assess the clinical effect (the so-called pharmacodynamics) of a drug at the target site, dose-response curves are generally used. Such curves, which are typically of a sigmoidal shape, describe the association between the drug concentration and a particular clinical effect. Knowing the dose-response relationship, a putative drug concentration at the site of action, the effect compartment E, can be calculated.

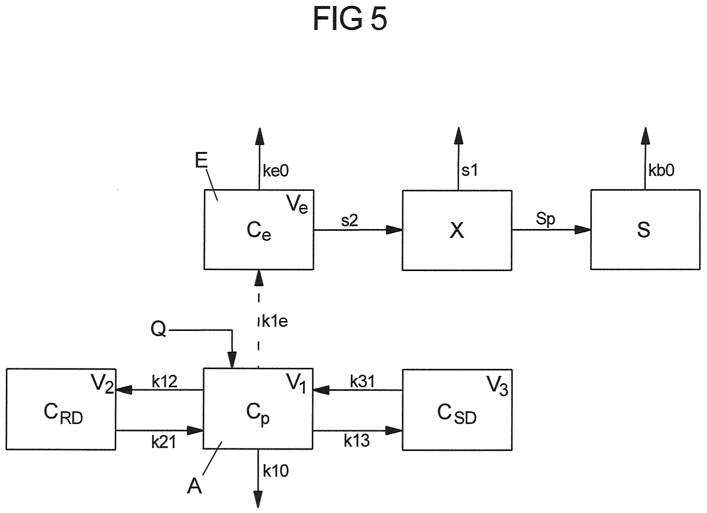

Fig. 5 shows a schematic diagram of another example of a PK/PD model, as it for example is described in WO 2017/190966 A1. In comparison to the model of Fig. 4, the model additionally comprises a remote compartment X and a BIS sensor compartment S, wherein

s1 and s2    represent constant transfer rate parameters between the remote compartment X and the effect compartment E,

$S_P$    represents a transfer rate coefficient between the remote compartment $X$ and the BIS sensor S, and

$k_{b0}$    represents the decay rate of the BIS index.

Clinically, $S_P$ can be regarded as a sensitivity value. The higher the value of $S_P$, the faster the drug's effect is achieved. High values of $S_P$ further lead to a small delay and a high responsiveness of the system.

The remote compartment X describes the delay between the drug's concentration in the effect-site compartment and its actual impact on the BIS value.

TCI models, e.g. for propofol, are known in the art. Recently introduced open-target-controlled infusion systems can be programmed with any pharmacokinetic model, and allow either plasma- or effect-site targeting. With effect-site targeting the goal is to achieve a user-defined target effect-site concentration as rapidly as possible, by manipulating the plasma concentration around the target. Currently systems are for example pre-programmed with a Marsh model (B. Marsh et al., "Pharmacokinetic model driven infusion of propofol in children" Br J Anaesth, 1991; 67, pages 41-48) or a Schnider model (Thomas W. Schnider et al., "The influence of method of administration and covariates on the pharmacokinetics of propofol in adult volunteers", Anesthesiology, 1998, 88(5) pages 1170-82).

The PK/PD model as shown in Fig. 5 can mathematically be described by the following set of (differential) equations.

Namely, the S compartment is described according to:

$$\dot{S} = \frac{s_p\, X}{\alpha_M + X} - k_{b0}S + OF$$

(Equation 1)

wherein

$s_P$    represents the drug sensitivity of the patient;

$\alpha_M$    represents the saturation parameter of the velocity of effect of a drug, e.g. an anesthetic agent such as propofol (i.e. the saturation of the drug receptors);

$k_{b0}$    represents the decay rate of the BIS index;

$OF$    represents the offset that can remain when no more drug is present in the patient body ;

$X$    represents a remote compartment; and

$S$    represents a sensor value of a BIS sensor.

The X compartment is described by:

$$\dot{X} = s_2 C_e - s_1 X$$

(Equation 2).

wherein

$s_1$ and $s_2$    represent constant transfer rate parameters between the remote compartment and the effect parameters;

$C_e$    represents the effect compartment concentration; and

$X$    represents a remote compartment.

The rapid equilibrating compartment $C_{RD}$ is described by:

$$\dot{C}_{RD} = -k_{21}C_{RD} + k_{12}C_p$$

(Equation 3)

wherein

$k_{12}$    is an elimination constant describing the drug distribution from the plasma compartment A1 in direction of rapid equilibrating compartment $C_{RD}$,

$k_{21}$    is an elimination constant describing the drug distribution from rapid equilibrating compartment $C_{RD}$ in direction of plasma compartment A1,

$C_{RD}$    represents the concentration in the rapid equilibrating compartment, and

$C_p$    represents the drug concentration in the plasma (blood) compartment.

The slow equilibrating compartment $C_{SD}$ is described by:

$$\dot{C}_{SD} = -k_{31}C_{SD} + k_{13}C_p$$

(Equation 4)

wherein

$k_{13}$    is an elimination constant describing the drug distribution from plasma compartment A1 in direction of slow equilibrating compartment $C_{SD}$,

$k_{31}$    is an elimination constant describing the drug distribution from slow equilibrating compartment $C_{SD}$ in direction of plasma compartment A1,

$C_{SD}$    represents the concentration in the slow equilibrating compartment; and

$C_p$    represents the drug concentration in the plasma (blood) compartment.

The effect compartment concentration $C_e$ is described by:

$$\dot{C}_e = -k_{e0}C_e + k_{1e}C_p$$

(Equation 5)

wherein

$k_{e0}$    defines a decay rate;

$k_{1e}$    describes a "virtual" constant rate transfer from plasma compartment A1 and the effect compartment E; and

$C_e$    represents the effect comportment concentration.

The blood concentration $C_p$ is described by:

$$\dot{C}_p = -(k_{10} + k_{12} + k_{13})C_p + k_{21}C_{RD} + k_{31}C_{SD}$$

(Equation 6)

wherein

$k_{10}$    represents the elimination constant of an applied drug from the body,

$k_{12}$    is an elimination constant describing the drug distribution from the plasma compartment A1 in direction of rapid equilibrating compartment $C_{RD}$,

$k_{21}$ is an elimination constant describing the drug distribution from rapid equilibrating compartment $C_{RD}$ in direction of plasma compartment A1,

$k_{13}$ is an elimination constant describing the drug distribution from plasma compartment A1 in direction of slow equilibrating compartment $C_{SD}$,

$k_{31}$ is an elimination constant describing the drug distribution from slow equilibrating compartment $C_{SD}$ in direction of plasma compartment A1,

$C_{RD}$ represents a rapid equilibrating compartment;

$C_{SD}$ represents a slow equilibrating compartment; and

$C_p$ represents the drug concentration in the plasma (blood) compartment.

Generally, the mathematical model, e.g. a PK/PD model as described above, during execution of an infusion operation is used to model the drug concentrations in certain body compartments of the patient, such that information about the drug distribution during the infusion operation is available for controlling the infusion operation using one or multiple infusion devices. The control herein is such that e.g. at the effect site, for example in the patient's brain, a drug concentration is established which is at or close to a desired target concentration, wherein for this the control device 2 (Figs. 1 and 2) controls infusion devices 31-33 such that a drug is infused to reach and maintain a drug concentration at the effect site at or close to the desired target concentration.

During execution of the infusion operation, the mathematical model may be tuned according to measurement values as obtained for example by a bio-signal monitor or from a sensor for measuring a drug concentration in the exhaled breath of a patient or the like. Using measurement information the mathematical model may be tuned according to actual concentration information by adjusting parameters of the model, for example transfer rate parameters or the like, such that the model correctly reflects the measured information and hence reliably predicts the drug concentrations in the different body compartments.

Referring now again to Fig. 1, each infusion device, in one embodiment, comprises a sensor device 311, 321, 331 which is configured to measure a value indicative of the pressure in the respective infusion line 310, 320, 330 connected to the infusion device 31, 32, 33. The sensor device 311, 321, 331 may for example have the shape of a pressure sensor for measuring a pressure in the infusion line or a force sensor for measuring a force acting on the infusion line. From the measurement of the sensor device 311, 321, 331 a value indicative of the pressure in the infusion line 310, 320, 330 may be derived and, in particular, a change in pressure in the infusion line 310, 320, 313 may be observed.

During an infusion operation, an occlusion of an infusion line 310, 320, 330 may occur, the occlusion preventing a fluid to flow through the infusion line 310, 320, 33 towards the patient P such that, from the time of the occurrence of the occlusion, no drug volume is infused into the patient P. By observing the pressure in the infusion line 310, 320, 330 using the sensor device 311, 321, 331 and by in particular observing whether the pressure in the infusion line 310, 320, 330 exceeds a feed predefined pressure threshold, the occlusion may be detected, and countermeasures to release the occlusion may be initiated by a user.

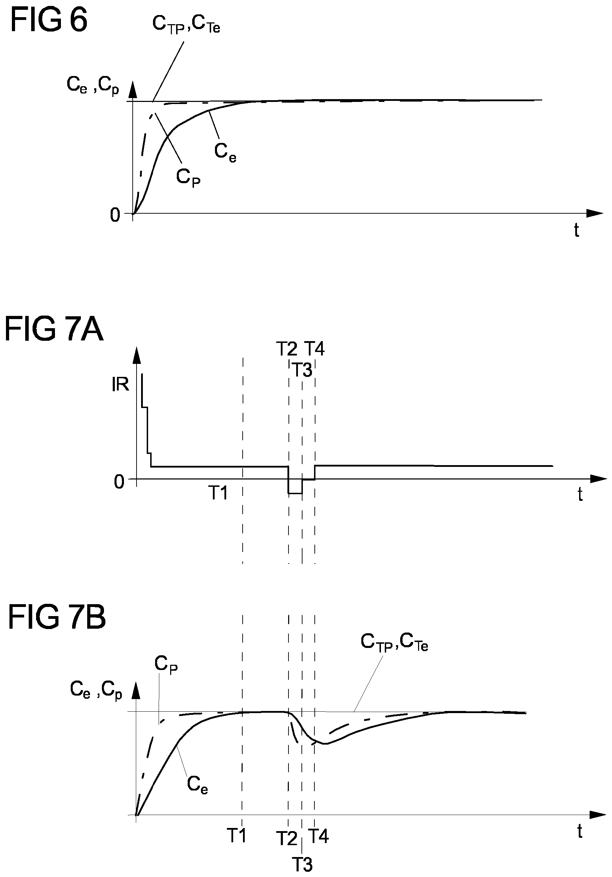

Referring now to Fig. 6, during execution of a target-controlled infusion protocol e.g. the plasma compartment concentration $C_p$ and/or the effect site concentration $C_e$ are controlled to converge towards a respective target concentration $C_{Tp}$, $C_{Te}$. In this way in particular an effect site concentration $C_e$ is established at or close to a desired target $C_{Te}$.

Fig. 6 herein shows the effect site concentration $C_e$ and the plasma concentration $C_P$ as computed by the mathematical model during the execution of the target-controlled infusion protocol.

Referring now to Figs. 7A, 7B, in case an occlusion in an infusion line 310, 320, 330 occurs, during the time span at which the occlusion is present no drug is infused to the patient. Herein, the occlusion can be detected by observing the pressure in the infusion line 310, 320, 330 using the sensor device 311, 321, 331, wherein however a significant gap between the time of the detection of the occlusion and the actual occurrence of the occlusion may exist, due to the fact that the occlusion is detected by observing whether the pressure in the infusion line exceeds a predefined threshold, wherein however the pressure in the infusion line rises, in case of an occlusion, with time until the pressure threshold is reached, the rise potentially taking several tens of minutes or even hours.

In the example of Figs. 7A, 7B, an occlusion may actually occur at a time T1. The occlusion however is only detected at time T2, substantially after the actual occurrence of the occlusion at time T1.

Once the occlusion is detected at time T2, the infusion device 31, 32, 33 is operated in reverse in order to reduce the pressure in the infusion line 310, 320, 330, indicated by the negative infusion rate IR between times T2 and T3 as shown in Fig. 7A. At time T3 the pressure in the infusion line 310, 320, 330 is released, and during a time period between times T3 and T4 it is checked whether the infusion may resume. At time T4 the infusion continues.

Fig. 7B shows the plasma concentration $C_p$ and the effect site concentration $C_e$ as computed with the mathematical model in case of the occlusion and by using information about the infused volume as derived from the infusion rate IR. Herein, it is visible that the mathematical model is fed with information about the occlusion only at time T2, but does not take into account that between times T1, T2 no drug actually reaches the patient. The computed plasma

concentration $C_p$ and effect site concentration $C_e$, as shown in Fig. 7B, hence are inaccurate, as the mathematical model does not consider the presence of the occlusion between times T1, T2.

For this reason, it is proposed to repeatedly store information derived from the mathematical model during execution of the target-controlled infusion protocol, and to keep the information in a memory 21 (see Fig. 2) of the control device 2. If in case of an occlusion it is detected that within the mathematical model it inaccurately has been assumed that a volume has been infused to the patient (without however reaching the patient due to the occlusion), it may be reverted to a prior state of the model, such that the actual drug distribution in the patient's body may be re-calculated while taking into account that during the occlusion no drug reaches the patient P.

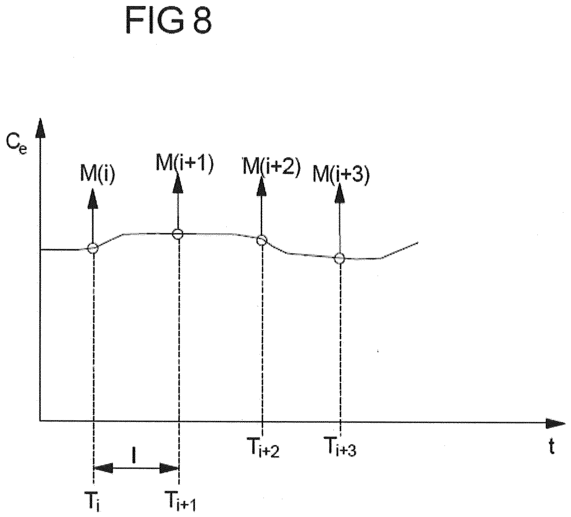

Referring now to Fig. 8, during execution of the target-controlled infusion protocol in one embodiment a set of parameters relating to the mathematical model is stored at different points in time $T_i...T_{i+3}$. The sets of parameters M(i)... M(i+3) may represent all of or a sub-set of the parameters as shown above in the system of equations representing the mathematical model, e.g. the computed drug concentrations in the different body compartments, the transfer rate constants, the decay rate constants, mass or volume information and the like at the different points in time $T_i...T_{i+3}$.

Herein, at a point in time $T_i...T_{i+3}$ the corresponding, current set of parameters M(i)... M(i+3) may be used to overwrite an earlier set of parameters, such that only a predefined number of sets of parameters, for example in between 20 to 100, for example 30 sets of parameters at a corresponding number of points in time $T_i...T_{i+3}$ are stored and kept in memory at any time.

At the different points in time $T_i...T_{i+3}$, hence, a snapshot of the model is stored in memory 21.

The information as derived from the mathematical model during execution of the target-controlled infusion protocol may be timestamped, such that the information is stored in memory together with an associated timestamp indicating the associated point in time $T_i...T_{i+3}$.

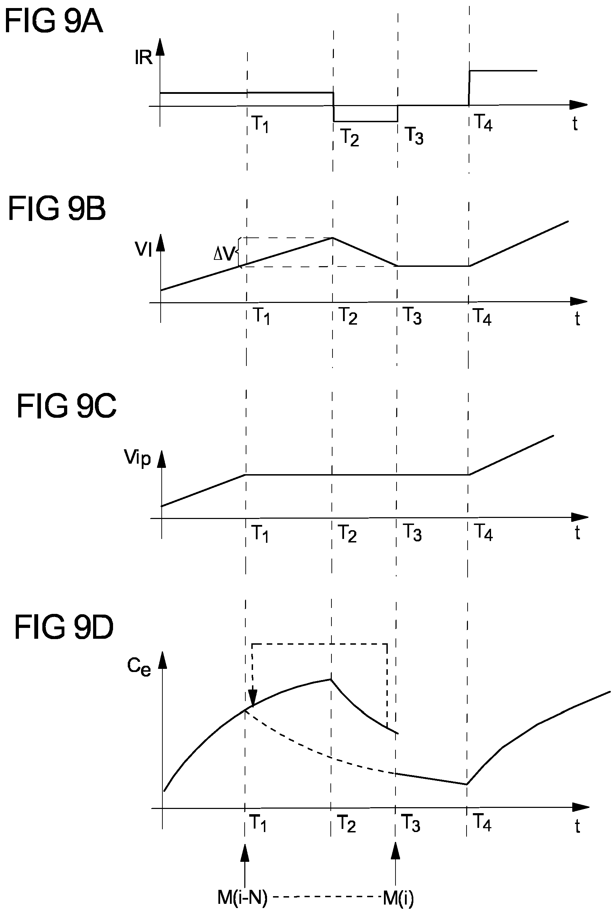

Referring now to Figs. 9A to 9D, using the stored sets of parameters at the different points in time the modeling of the drug distribution within the patient may become more accurate in case of an occlusion. Fig. 9A herein shows the infusion rate IR as applied by the infusion device 31, 32, 33. Fig. 9B shows the (total) infused volume as transported (pumped) by the infusion device 31, 32, 33. Fig. 9C shows the (total) infused volume which reaches the patient. Fig. 9D shows the computed effect site concentration $C_e$, when applying a correction scheme in case of the detection of an occlusion.

In the example of Figs. 9A to 9D, at time T1 an occlusion occurs. The occlusion however is detected, by observing the pressure in the infusion line 310, 320, 330, only at time T2, such that between times T1 and T2 the infusion rate IR (Fig. 9A) of the infusion device 31, 32, 33 remains unchanged, and the infused volume VI as transported by the infusion device 31, 32, 33 keeps steadily increasing, according to the constant infusion rate IR.

However, at the time T1 of occurrence of the occlusion no drug any longer reaches the patient, such that the volume VIp actually infused into the patient P does not change between times T1 and T2.

At time T2 the occlusion is detected. Following the detection of the occlusion, a pressure release process is started, in which the volume which is transported by the infusion device 31, 32, 33 between times T1 and T2 (denoted herein as transport volume ΔV) is pumped in reverse by operating the infusion device 31, 32, 33 between times T2 and T3 in a reverse pumping direction with a negative infusion rate IR (Fig. 9A). The total infused volume VI according to Fig. 9B hence is reduced by the transport volume ΔV, such that at time T3 the total infused volume VI as transported by the infusion device 31, 32, 33 is again at least approximately equal to the infused volume VI at the occurrence of the occlusion at time T1.

As during the pressure release process the occlusion in the line 310, 320, 330 is maintained to avoid a bolus due to the excess pressure in the infusion line 310, 320, 330, the infused volume VIp reaching the patient does not change between times T2 and T3 (Fig. 9C).

The pressure release process may for example be carried out as it is described in EP 3 228 343 A1.

At time T3 the pressure in the occluded infusion line 310, 320, 330 is released, and the occlusion hence can be released. Between times T3 and T4 it is checked (at a Zero infusion rate IR) whether the infusion operation may resume, and at time T4 the infusion is continued with a positive infusion rate IR (Fig. 9A), leading to an increase in the volume VI transported by the infusion device (Fig. 9B) and the volume VIp reaching the patient (Fig. 9C).

Fig. 9D shows the computed effect site concentration $C_e$. Herein, as indicated by the solid line, the mathematical model computes the effect site concentration $C_e$ at first without knowledge about the occlusion and while assuming that between times T1 and T2 a drug volume is infused into the patient P. At time T2 the occlusion is detected, and the infusion rate IR is reversed (Fig. 9A), causing a decay in the effect site concentration $C_e$, as visible by the solid line in between times T2 and T3 in Fig. 9D. As this modeling of the effect site concentration Ce falsely assumes a volume having been infused into the patient between times T1 and T2, the effect site concentration $C_e$ as indicated by the solid line between times T1 and T3 is inaccurate.

In order to provide for an accurate modeling of the drug distribution within the patient P, it hence is reverted to information stored in relation to the mathematical model at a previous time.

Namely, it is searched for that set of parameters M(i-N) at which the infused volume VI is equal to or smaller than the infused volume at the end of the pressure release process at time T3. It hence is searched for that set of parameters M(i-N) at which the infused volume VI at the time T2 of detection of the occlusion is reduced by the transport volume ΔV, which is transported by the infusion device 31, 32, 33 during the presence of the occlusion and which subsequently is reversed by the pressure release process.

For searching for the set of parameters M(i-N) corresponding to the infused volume VI equal to or smaller than the infused volume VI at time T3, the sets of parameters may be successively evaluated by comparing a stored value of the infused volume VI of the respective set of parameters to the value of the infused volume VI at the time T3, starting e.g. with the most recent available set of parameters.

Once the set of parameters M(i-N) is identified in which the stored value of the infused volume VI has become equal to or smaller than the infused volume VI at the time T3 of the pressure release, the particular set of parameters M(i-N) is chosen and used in the mathematical model, such that the drug distribution in the patient's body is modeled anew starting with the point in time $T_{i-N}$ corresponding to the set of parameters M(i-N). The point in time $T_{i-N}$ herein approximately is equal to the time T1 of the actual occurrence of the occlusion.

It hence is reverted to the state of the mathematical model approximately at time T1, i.e., at the time of occurrence of the occlusion. By using this set of parameters M(i-N) in the mathematical model, the drug distribution in the patient's body may correctly be re-calculated in between times T1 and T3, by assuming that during the time period between times T1 and T3 no drug is infused into and reaches the patient P. This is indicated by the dashed line between times T1 and T3 in Fig. 9D.

At time T3, i.e., at the time at which the occlusion is released, an accurate estimation of the drug distribution, as illustrated by the effect site concentration $C_e$ in Fig. 9D, thus is available. Following time T3, hence, the further drug distribution and concentrations within the patient's body may be accurately modelled using the mathematical model, as indicated by the solid line starting at time T3 in Fig. 9D.

The idea of the invention is not limited to the embodiments described above, but may be implemented in a different fashion.

A target-controlled infusion may generally be used for performing an anesthesia operation on a patient, but may also be employed for infusing drugs to a patient to achieve a therapeutic action.

An infusion operation herein may involve one or multiple drugs administered using one or multiple infusion devices

**List of Reference Numerals**

| | |
|---|---|
| 1 | Rack |
| 2 | Control device |
| 20 | Measurement device |
| 21 | Memory |
| 31, 32, 33 | Infusion device |
| 310, 320, 330 | Line |
| 311, 321, 331 | Sensor device (pressure sensor) |
| 4 | Ventilation device |
| 40 | Mouth piece |
| 400 | Line |
| 5 | Bio-signal monitor |
| 50 | Electrodes |
| 500 | Line |
| 6 | Display device |
| 7 | Monitor device |
| A1-A5 | Compartments |
| $C_e$ | Effect site concentration |
| $C_p$ | Concentration of plasma compartment |
| $C_{RD}$ | Concentration of rapid equilibrating compartment |
| $C_{SD}$ | Concentration of slow equilibrating compartment |
| $C_{Tp}$, $C_{Te}$ | Target concentration |
| D | Drug dosage |
| ΔV | Transported volume |
| E | Effect site compartment |
| I | Interval |

| IR | Infusion rate |
|---|---|
| k12, k21, k31, k13, k1e, k10 | Parameters |
| kb0 | Decay rate |
| P | Patient |
| S | Sensor value |
| s1, s2 | Transfer rate parameter |
| T1-T4 | Point in time |
| $T_i...T_{i+3}$ | Point in time |
| Q | Drug |
| U | Operator (practitioner) |
| $V_1$-$V_3$, $V_e$ | Volume |
| VI | Infused volume transported by pump |
| $VI_P$ | Infused volume to patient |
| X | Remote compartment |

**Claims**

1. A system for controlling a target-controlled infusion for administering a drug to a patient (P), comprising:

   at least one infusion device (31-33) for administering a drug to the patient (P), the at least one infusion device (31, 33) comprising a sensor device (311, 321, 331) for measuring a value indicative of a pressure in an infusion line (310, 320, 330) connected to the at least one infusion device (31-33) for delivering said drug to the patient (P); a control device (2) is configured to control operation of the at least one infusion device (31-33) in order to establish a drug concentration within the patient (P) based on a target concentration, wherein the control device (2) is configured to execute a target-controlled infusion protocol using a mathematical model modelling a drug distribution in the patient's body for controlling operation of the at least one infusion device (31-33);
   wherein the control device (2) is further configured to detect an occlusion in the infusion line (310, 320, 330) connected to the at least one infusion device (31-33) based on said value measured by the sensor device (311, 321, 331);
   **characterized in that** the control device (2) is configured to store, at each of a multiplicity of points in time ($T_i...T_{i+3}$) during execution of said target-controlled infusion protocol, a set of parameters derived from said mathematical model in a memory (21), wherein the control device (2) is configured, in case an occlusion is detected, to use the set of parameters of a point in time ($T_i...T_{i+3}$) prior to the detection of the occlusion in the mathematical model to model the drug distribution in the patient's body based on the set of parameters of the point in time ($T_i...T_{i+3}$) prior to the detection of the occlusion.

2. The system according to claim 1, **characterized in that** the control device (2) is configured to associate the set of parameters at each point in time ($T_i...T_{i+3}$) with a timestamp indicative of the point in time ($T_i...T_{i+3}$), and to store the set of parameters together with the associated timestamp in the memory (21).

3. The system according to claim 1 or 2, **characterized in that** the multiplicity of points in time ($T_i...T_{i+3}$) are equidistantly spaced apart at predefined time intervals (I).

4. The system according to claim 1 or 2, **characterized in that** the multiplicity of points in time ($T_i...T_{i+3}$) are associated with a predefined change in an infused volume.

5. The system according to one of the preceding claims, **characterized in that** the control device (2) is configured, at the time of detection of the occlusion, to initiate a pressure release process to reduce a pressure within the infusion line (310, 320, 330).

6. The system according to claim 5, **characterized in that** the control device (2) is configured, during the pressure release process, to transport a reduction volume in an upstream direction in order to reduce an amount of an infused volume.

7. The system according to claim 6, **characterized in that** the control device (2) is configured to identify the set of parameters of the point in time ($T_i...T_{i+3}$) prior to the detection of the occlusion based on the reduction volume.

8. The system according to claim 6 or 7, **characterized in that** the control device (2) is configured to identify the set of parameters of the point in time ($T_i$...$T_{i+3}$) prior to the detection of the occlusion by comparing a value indicative of the infused volume as stored in the sets of parameters of the multiplicity of points in time ($T_i$...$T_{i+3}$) to a value indicative of the infused volume as a result of the pressure release process.

9. The system according to claim 8, **characterized in that** the control device (2) is configured to compare the value indicative of the infused volume as stored in the set of parameters to the value indicative of the infused volume as a result of the pressure release process starting with the set of parameters at a most recent point in time ($T_i$...$T_{i+3}$).

10. The system according to claim 8 or 9, **characterized in that** the control device (2) is configured to use the set of parameters of a point in time ($T_i$...$T_{i+3}$) prior to the detection of the occlusion at which the value indicative of the infused volume as stored in the set of parameters is equal to or smaller than the value indicative of the infused volume as a result of the pressure release process.

11. The system according to one of claims 5 to 10, **characterized in that** the control device (2) is configured, by using the set of parameters of said point in time ($T_i$...$T_{i+3}$) prior to the detection of the occlusion in the mathematical model, to model the drug distribution in the patient's body at the time of concluding the pressure release process.

12. The system according to one of the preceding claims, **characterized in** the system according to one of the preceding claims, **characterized in that** the mathematical model is a pharmacokinetic/pharmacodynamic model.

13. The system according to one of the preceding claims, **characterized in** the system according to one of the preceding claims, **characterized in that** the mathematical model models a drug concentration in a multiplicity of compartments of the patient (P) during execution of the target-controlled infusion protocol.

14. The system according to claim 13, **characterized in that** the mathematical model is described by a multiplicity of parameters, wherein the control device (2) is configured to adjust at least a subgroup of said multiplicity of parameters during execution of the target-controlled infusion protocol according to measurement values relating to a drug concentration distribution in the patient.

**Patentansprüche**

1. System zur Steuerung einer zielgesteuerten Infusion zur Verabreichung eines Medikaments an einen Patienten (P), das Folgendes umfasst:

   mindestens eine Infusionsvorrichtung (31-33) zur Verabreichung eines Medikaments an den Patienten (P), wobei die mindestens eine Infusionsvorrichtung (31-33) eine Sensorvorrichtung (311, 321, 331) zur Messung eines Wertes, der einen Druck in einer Infusionsleitung (310, 320, 330) anzeigt, die mit der mindestens einen Infusionsvorrichtung (31-33) zur Abgabe des Medikaments an den Patienten (P) verbunden ist, umfasst;
   eine Steuervorrichtung (2), die dazu konfiguriert ist, den Betrieb der mindestens einen Infusionsvorrichtung (31-33) zu steuern, um basierend auf einer Zielkonzentration eine Medikamentenkonzentration in dem Patienten (P) zu etablieren, wobei die Steuervorrichtung (2) dazu konfiguriert ist, unter Verwendung eines mathematischen Modells, das eine Medikamentenverteilung im Körper des Patienten modelliert, zum Steuern des Betriebs der mindestens einen Infusionsvorrichtung (31-33) ein Protokoll der zielgesteuerten Infusion auszuführen;
   wobei die Steuervorrichtung (2) ferner dazu konfiguriert ist, einen Verschluss in der Infusionsleitung (310, 320, 330), die mit der mindestens einen Infusionsvorrichtung (31-33) verbunden ist, basierend auf dem von der Sensorvorrichtung (311, 321, 331) gemessenen Wert zu detektieren;
   **dadurch gekennzeichnet, dass** die Steuervorrichtung (2) dazu konfiguriert ist, an jedem einer Vielzahl von Zeitpunkten ($T_i$...$T_{i+3}$) während der Ausführung des Protokolls der zielgesteuerten Infusion einen Satz von dem mathematischen Modell abgeleiteter Parameter in einem Speicher (21) zu speichern, wobei die Steuervorrichtung (2) dazu konfiguriert ist, im Falle eines detektierten Verschlusses den Satz Parameter zu einem Zeitpunkt ($T_i$...$T_{i+3}$) vor der Detektion des Verschlusses in dem mathematischen Modell zu verwenden, um die Medikamentenverteilung im Körper des Patienten basierend auf dem Satz Parameter des Zeitpunktes ($T_i$...$T_{i+3}$) vor der Detektion des Verschlusses zu modellieren.

2. System nach Anspruch 1, **dadurch gekennzeichnet, dass** die Steuervorrichtung (2) dazu konfiguriert ist, den Satz Parameter zu jedem Zeitpunkt ($T_i$...$T_{i+3}$) einem den Zeitpunkt ($T_i$...$T_{i+3}$) anzeigenden Zeitstempel zuzuordnen und den Satz Parameter zusammen mit dem zugeordneten Zeitstempel im Speicher (21) zu speichern.

3. System nach Anspruch 1 oder 2, **dadurch gekennzeichnet, dass** die Vielzahl der Zeitpunkte ($T_i$...$T_{i+3}$) in zuvor definierten Zeitabständen (I) abstandsgleich beabstandet sind.

4. System nach Anspruch 1 oder 2, **dadurch gekennzeichnet, dass** die Vielzahl der Zeitpunkte ($T_i$...$T_{i+3}$) einer zuvor definierten Änderung eines Infusionsvolumens zugeordnet sind.

5. System nach einem der vorhergehenden Ansprüche, **dadurch gekennzeichnet, dass** die Steuervorrichtung (2) dazu konfiguriert ist, zum Zeitpunkt der Detektion des Verschlusses einen Druckentlastungsprozess einzuleiten, um einen Druck innerhalb der Infusionsleitung (310, 320, 330) zu reduzieren.

6. System nach Anspruch 5, **dadurch gekennzeichnet, dass** die Steuervorrichtung (2) dazu konfiguriert ist, während des Druckentlastungsprozesses ein Reduktionsvolumen in einer Stromaufwärtsrichtung zu transportieren, um eine Menge eines Infusionsvolumens zu reduzieren.

7. System nach Anspruch 6, **dadurch gekennzeichnet, dass** die Steuervorrichtung (2) dazu konfiguriert ist, den Satz Parameter des Zeitpunkts ($T_i$...$T_{i+3}$) vor der Detektion des Verschlusses basierend auf dem Reduktionsvolumen zu identifizieren.

8. System nach Anspruch 6 oder 7, **dadurch gekennzeichnet, dass** die Steuervorrichtung (2) dazu konfiguriert ist, den Satz Parameter des Zeitpunkts ($T_i$...$T_{i+3}$) vor der Detektion des Verschlusses durch Vergleichen eines Wertes, der das Infusionsvolumen wie in den Sätzen von Parametern der Vielzahl von Zeitpunkten ($T_i$...$T_{i+3}$) gespeichert anzeigt, mit einem Wert, der das Infusionsvolumen als Ergebnis des Druckentlastungsprozesses anzeigt, zu identifizieren.

9. System nach Anspruch 8, **dadurch gekennzeichnet, dass** die Steuervorrichtung (2) dazu konfiguriert ist, den Wert, der das Infusionsvolumen wie in dem Satz Parameter gespeichert anzeigt, mit dem Wert, der das Infusionsvolumen als Ergebnis des Druckentlastungsprozesses anzeigt, zu vergleichen, beginnend mit dem Satz Parameter zum aktuellsten Zeitpunkt ($T_i$...$T_{i+3}$).

10. System nach Anspruch 8 oder 9, **dadurch gekennzeichnet, dass** die Steuervorrichtung (2) dazu konfiguriert ist, den Satz Parameter eines Zeitpunkts ($T_i$...$T_{i+3}$) vor der Detektion des Verschlusses zu verwenden, bei dem der Wert, der das Infusionsvolumen wie in dem Satz Parameter gespeichert anzeigt, gleich dem oder kleiner als der Wert ist, der das Infusionsvolumen als Ergebnis des Druckentlastungsprozesses anzeigt.

11. System nach einem der Ansprüche 5 bis 10, **dadurch gekennzeichnet, dass** die Steuervorrichtung (2) dazu konfiguriert ist, durch Verwendung des Satzes Parameter des Zeitpunkts ($T_i$...$T_{i+3}$) vor der Detektion des Verschlusses in dem mathematischen Modell die Medikamentenverteilung im Körper des Patienten zum Zeitpunkt der Beendigung des Druckentlastungsprozesses zu modellieren.

12. System nach einem der vorhergehenden Ansprüche, **gekennzeichnet durch** das System nach einem der vorhergehenden Ansprüche, **dadurch gekennzeichnet,** dass das mathematische Modell ein pharmakokinetisches/pharmakodynamisches Modell ist.

13. System nach einem der vorhergehenden Ansprüche, **gekennzeichnet durch** das System nach einem der vorhergehenden Ansprüche, **dadurch gekennzeichnet,** dass das mathematische Modell eine Arzneimittelkonzentration in einer Vielzahl von Kompartimenten des Patienten (P) während der Ausführung des Protokolls der zielgesteuerten Infusion modelliert.

14. System nach Anspruch 13, **dadurch gekennzeichnet, dass** das mathematische Modell durch eine Vielzahl von Parametern beschrieben wird, wobei die Steuervorrichtung (2) dazu konfiguriert ist, mindestens eine Untergruppe der Vielzahl von Parametern während der Ausführung des Protokolls der zielgesteuerten Infusion gemäß Messwerten bezüglich einer Medikamentenkonzentrationsverteilung in dem Patienten anzupassen.

**Revendications**

1. Système de commande d'une perfusion commandée par cible pour l'administration d'un médicament à un patient (P), comprenant :

au moins un dispositif de perfusion (31 à 33) pour l'administration d'un médicament au patient (P), l'au moins un dispositif de perfusion (31, 33) comprenant un dispositif capteur (311, 321, 331) pour la mesure d'une valeur indiquant une pression dans un tube de perfusion (310, 320, 330) raccordé à l'au moins un dispositif de perfusion (31 à 33) pour la délivrance dudit médicament au patient (P) ;

un dispositif de commande (2) est conçu pour commander le fonctionnement de l'au moins un dispositif de perfusion (31 à 33) afin d'établir une concentration de médicament au sein du patient (P) en fonction d'une concentration cible, dans lequel le dispositif de commande (2) est conçu pour exécuter un protocole de perfusion commandé par cible à l'aide d'un modèle mathématique modélisant une distribution de médicament dans le corps du patient pour commander le fonctionnement de l'au moins un dispositif de perfusion (31 à 33) ;

dans lequel le dispositif de commande (2) est en outre conçu pour détecter une occlusion dans le tube de perfusion (310, 320, 330) raccordé à l'au moins un dispositif de perfusion (31 à 33) en fonction de ladite valeur mesurée par le dispositif capteur (311, 321, 331) ;

**caractérisé en ce que** le dispositif de commande (2) est conçu pour stocker, au niveau de chacun d'une multiplicité de points dans le temps ($T_i...T_{i+3}$) au cours de l'exécution dudit protocole de perfusion commandé par cible, un ensemble de paramètres dérivés dudit modèle mathématique dans une mémoire (21), dans lequel le dispositif de commande (2) est conçu, dans un cas où une occlusion est détectée, pour utiliser l'ensemble de paramètres d'un point dans le temps ($T_i...T_{i+3}$) avant la détection de l'occlusion dans le modèle mathématique pour modéliser la distribution de médicament dans le corps du patient en fonction de l'ensemble des paramètres du point dans le temps ($T_i...T_{i+3}$) avant la détection de l'occlusion.

2. Système selon la revendication 1, **caractérisé en ce que** le dispositif de commande (2) est conçu pour associer l'ensemble de paramètres à chaque point dans le temps ($T_i...T_{i+3}$) à un horodatage indiquant le point dans le temps ($T_i...T_{i+3}$), et pour stocker l'ensemble de paramètres conjointement avec l'horodatage associé dans la mémoire (21).

3. Système selon la revendication 1 ou 2, **caractérisé en ce que** la multiplicité des points dans le temps ($T_i...T_{i+3}$) sont espacés de manière équidistante à des intervalles de temps prédéfinis (I).

4. Système selon la revendication 1 ou 2, **caractérisé en ce que** la multiplicité des points dans le temps ($T_i...T_{i+3}$) sont associés à un changement prédéfini dans un volume perfusé.

5. Système selon l'une des revendications précédentes, **caractérisé en ce que** le dispositif de commande (2) est conçu, au temps de la détection de l'occlusion, pour lancer un processus de libération de pression pour réduire une pression au sein du tube de perfusion (310, 320, 330).

6. Système selon la revendication 5, **caractérisé en ce que** le dispositif de commande (2) est conçu, pendant le processus de libération de pression, pour transporter un volume de réduction dans une direction amont afin de réduire une quantité d'un volume perfusé.

7. Système selon la revendication 6, **caractérisé en ce que** le dispositif de commande (2) est conçu pour identifier l'ensemble de paramètres du point dans le temps ($T_i...T_{i+3}$) avant la détection de l'occlusion en fonction du volume de réduction.

8. Système selon la revendication 6 ou 7, **caractérisé en ce que** le dispositif de commande (2) est conçu pour identifier l'ensemble de paramètres du point dans le temps ($T_i...T_{i+3}$) avant la détection de l'occlusion en comparant une valeur indiquant le volume perfusé telle qu'elle est stockée dans les ensembles de paramètres de la multiplicité de points dans le temps ($T_i...T_{i+3}$) à une valeur indiquant le volume perfusé résultant du processus de libération de pression.

9. Système selon la revendication 8, **caractérisé en ce que** le dispositif de commande (2) est conçu pour comparer la valeur indiquant le volume perfusé telle qu'elle est stockée dans l'ensemble de paramètres à la valeur indiquant le volume perfusé résultant du processus de libération de pression commençant avec l'ensemble de paramètres au niveau d'un point dans le temps ($T_i...T_{i+3}$) le plus récent.

10. Système selon la revendication 8 ou 9, **caractérisé en ce que** le dispositif de commande (2) est conçu pour utiliser l'ensemble de paramètres d'un point dans le temps ($T_i...T_{i+3}$) avant la détection de l'occlusion au niveau duquel la valeur indiquant le volume perfusé telle qu'elle est stockée dans l'ensemble de paramètres est égale ou inférieure à la valeur indiquant le volume perfusé résultant du processus de libération de pression.

11. Système selon l'une des revendications 5 à 10, **caractérisé en ce que** le dispositif de commande (2) est conçu, à l'aide de l'ensemble de paramètres dudit point dans le temps ($T_i$...$T_{i+3}$) avant la détection de l'occlusion dans le modèle mathématique, pour modéliser la distribution de médicament dans le corps du patient au temps de la conclusion du processus de libération de pression.

12. Système selon l'une des revendications précédentes, **caractérisé par** le système selon l'une des revendications précédentes, **caractérisé en ce que** le modèle mathématique est un modèle pharmacocinétique/pharmacodynamique.

13. Système selon l'une des revendications précédentes, **caractérisé par** le système selon l'une des revendications précédentes, **caractérisé en ce que** le modèle mathématique modélise une concentration de médicament dans une multiplicité de compartiments du patient (P) pendant l'exécution du protocole de perfusion commandé par cible.

14. Système selon la revendication 13, **caractérisé en ce que** le modèle mathématique est décrit par une multiplicité de paramètres, dans lequel le dispositif de commande (2) est conçu pour ajuster au moins un sous-groupe de ladite multiplicité de paramètres pendant l'exécution du protocole de perfusion commandé par cible selon des valeurs de mesure relatives à une distribution de concentration de médicament dans le patient.

# FIG 1

FIG 2

EP 4 423 762 B1

FIG 3

# FIG 4

# FIG 5

# FIG 6

# FIG 7A

# FIG 7B

FIG 8

FIG 9A

FIG 9B

FIG 9C

FIG 9D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1418976 B1
- WO 2016160321 A1
- WO 2017190966 A1
- US 10842932 B1
- US 2018085520 A1
- EP 3228343 A1

### Non-patent literature cited in the description

- **B. MARSH et al.** Pharmacokinetic model driven infusion of propofol in children. *Br J Anaesth*, 1991, vol. 67, 41-48
- **THOMAS W. SCHNIDER et al.** The influence of method of administration and covariates on the pharmacokinetics of propofol in adult volunteers. *Anesthesiology*, 1998, vol. 88 (5), 1170-82